(19) Europäisches Patentamt
European Patent Office
Office européen des brevets

(11) Veröffentlichungsnummer: **0 467 369 A2**

(12) # EUROPÄISCHE PATENTANMELDUNG

(21) Anmeldenummer: **91112048.3**

(51) Int. Cl.5: **A61K 31/63**

(22) Anmeldetag: **18.07.91**

(30) Priorität: **20.07.90 DE 4023086**

(43) Veröffentlichungstag der Anmeldung: **22.01.92 Patentblatt 92/04**

(84) Benannte Vertragsstaaten: **AT BE CH DE DK ES FR GB GR IT LI LU NL SE**

(71) Anmelder: **HOECHST AKTIENGESELLSCHAFT**
**Postfach 80 03 20**
**W-6230 Frankfurt am Main 80(DE)**

(72) Erfinder: **Bianco, Sebastiano, Prof. Dr.**
**Via Cottolengo 42**
**I-20143 Milano(IT)**

(54) **Verwendung von Furosemid zur Behandlung von allergischer Konjunkfivitis.**

(57) Furosemid eignet sich hervorragend zum Behandeln der allergischen Konjunktivitis. Beschrieben wird auch die Verwendung von Furosemid zur Herstellung eines Medikaments zur Behandlung bzw. Verhütung allergischer Konjunktivitis.

Fig. 1

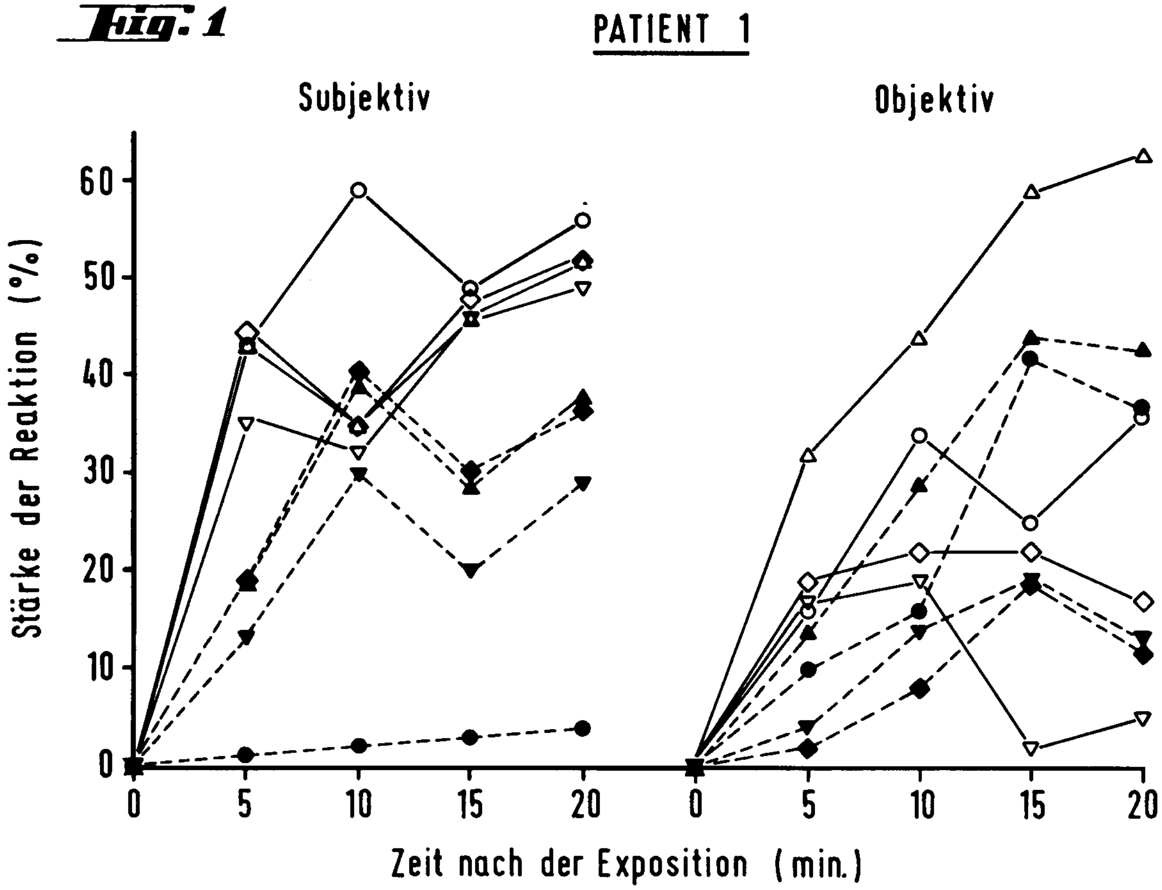


EP 0 467 369 A2

Die Erfindung betrifft eine Methode zur Behandlung allergischer Konjunktivitis.

Furosemid ist ein wohlbekanntes Diuretikum

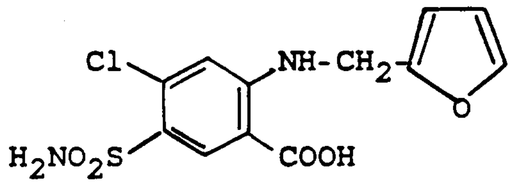

und ist außerdem als Medikament zur Verhütung bzw. Behandlung von Asthma bekannt (vgl. The Lancet, 30. Juli 1988, S.252).

Wir haben nun gefunden, daß Furosemid ein wertvolles Medikament zur Behandlung von allergischer Konjunktivitis ist, was weder erwartet noch durch den Stand der Technik nahegelegt worden war. Die Erfindung betrifft weiterhin eine Methode zur Behandlung bzw. Verhütung allergischer Konjunktivitis, die die Applikation einer geeigneten Furosemidmenge in den Fornix conjunctivae des Auges beinhaltet, sowie die Verwendung von Furosemid zu diesem Zweck und die Verwendung von Furosemid zur Herstellung eines Medikaments für die Behandlung bzw. Verhütung allergischer Konjunktivitis.

## Material und Methoden

**Patienten:** 10 Patienten mit einer klinischen Vorgeschichte von saisonaler allergischer Konjunktivitis mit oder ohne Rhinitis oder Asthma und mit einer positiven Hautreaktion auf Graspollen wurden untersucht. Die klinischen Merkmale der Patienten sind in Tabelle I aufgeführt. Alle Probanden waren nicht behandelt worden, und die Experimente wurden vollständig außerhalb der Pollenzeit ausgeführt.

**Methoden:** Der allergische Konjunktivaltest wurde ausgeführt wie bei Bonini S, Bonini S, Berruto A, Tomassini M, Carlesimo S, Bucci MG, Balsano F. Conjunctival provocation test as a model for the study of allergy and inflammation in humans [Der konjunktivale Reizungstest als ein Modell für die Untersuchung von Allergie und Entzündung beim Menschen]. Int Arch Allergy Appll Immunol 1989, 88:144-148 beschrieben.

Kurzbeschreibung: An einem Vorbereitungstag wurde eine Titration des Hautempfindlichkeitsendpunktes unter Verwendung von ansteigenden Verdünnungen (Verdünnungsfaktor 3,2) des Allergens in physiologischer Kochsalzlösung mit 0,03% humanem Serumalbumin (im Bereich von 10.000 bis 3 RAST-Einheiten $ml^{-1}$, Frazioni alfa, Bayropharm Italiana, Mailand, Italien) am Unterarm durchgeführt. Die niedrigste Dosis, die nach 15 bis 20 Minuten zu einer Quaddelbildung mit einem Durchmesser von mindestens 2 mm im Vergleich zur Negativkontrolle führte, wurde dann als erste Dosis für den Konjunktivaltest des Allergens verwendet.

Zur Konjunktivalexposition wurde der Patient mit nach hinten geneigtem aufrechten Kopf positioniert, und ein Tropfen Allergen bzw. Kontrollösung wurde in den unteren Fornix conjunctivae eines Auges appliziert. Subjektive und objektive Symptome wurden vom Patienten und vom Versuchsleiter 5, 10, 15, 20 und 30 Minuten nach der Reizung auf einer kontinuierlichen visuellen Analogskala zwischen 0 (keine Symptome) und 100% (maximal erträgliche Symptome) aufgenommen. Subjektive Symptome wurden vom Patienten aufgenommen und beinhalteten Jucken, Brennen, Tränenbildung und Photophobie. Vom Versuchsleiter aufgenommene objektive Symptome waren Erythem, Ödem, Bindehautsgefäßstauungen und Tränenbildung. In einigen Fällen wurden zum Nachweis der objektiven Beobachtungen nach 20 Minuten photographische Makroaufnahmen mit einem Aufnahmemaßstab von 1:1 angefertigt.

Wenn die Reaktion nach 20 Minuten einen Wert von 50 % oder mehr bei mindestens 2 der Untersuchungsparameter nicht erreicht hatte, wurde der Test am kontralateralen Auge mit der nächsten, höher konzentrierten Allergenkonzentration wiederholt. Alle Patienten sprachen auf die erste bzw. die zweite Allergendosis an. Diese einen Reiz hervorrufende Dosis wurde dann für die nachfolgenden experimentellen Konjunktivalexpositionen eingesetzt.

**Versuchskonzept:** Nachdem die Empfindlichkeit der Bindehaut im Vorversuch bestimmt worden war, führte jeder Patient zwei Konjunktivalexpositionen mit einem einwöchentlichen Abstand durch, und zwar nach einer Vorbehandlung mit 4 Tropfen 10 mg/ml Furosemid (®Lasix, Hoechst) bzw. Plazebo gemäß einem randomisierten Doppelblindversuchsprotokoll. 2 Tropfen der Behandlungslösung wurden 5 Minuten vor dem Test an jedem Auge verabreicht, 2 unmittelbar vor der Allergenexposition mit einer einzelnen Reizdosis. Der Test wurde dann wie für den Vorversuch beschrieben ausgeführt.

**Statistische Auswertung:** Die mit der Analogskala erhaltenen Daten wurden mit nichtparametrischen Standardmethoden für nicht verbundene Stichproben analysiert, obwohl sie in einer kontinuierlichen Skala aufgenommen wurden, da Linearität nicht vorausgesetzt werden konnte (Maatthews DE, Farewell WT. Using and understanding medical statistics (Medizinische Statistik anwenden und verstehen], 2. Auflage. Karger, Basel, 1988). Für multiple Vergleiche wurde Varianzanalyse verwendet. Ein Wert von 0,05 für p im zweiseitigen Test wurde als signifikant angesehen.

**Ergebnisse**

Nach der Plazebobehandlung reagierten sensitive Probanden mit Jucken, Brennen, vermehrter Gefäßzeichnung, Erythem und Tränenbildung auf den Augenreiz, während ein brennendes Gefühl in den Augen, Photophobie und Ödem unregelmäßiger verteilt waren. Zwei Patienten gaben außerdem Niesen und "laufende" Nase als Befund an, was wahrscheinlich auf ein Durchsickern von Allergen durch den Tränen-Nasen-Gang in die Nase zurückzuführen ist. Es wurde ein erhebliches Ausmaß an Schwankungen der Intensität und des zeitlichen Verlaufs der subjektiven bzw. der objektiven Parameter der Reaktion auf die Konjunktivalexposition bei den unterschiedlichen Probanden beoachtet, wie in Abbildung 1 bis 10 gezeigt wird, wo die für jeden Probanden während der Plazebo- bzw. Furosemidexposition erhaltenen Werte aufgeführt sind. Die beschreibenden statistischen Parameter der subjektiven Werte sind in Tabelle II aufgeführt, die der objektiven Werte in Tabelle III. Alle objektiven und subjektiven Symptome lagen normalerweise 5 Minuten nach der Exposition vor, wobei keine signifikanten Schwankungen an den verschiedenen Zeitpunkten bis zu 20 Minuten auftraten, obwohl es schien, daß die subjektiven Symptome zu einem frühen Zeitpunkt deutlicher waren, während die objektiven Symptome während der Exposition eher allmählich anstiegen. Demgegenüber schienen die subjektiven Werte für Tränenbildung später als die objektiven Werte für das gleiche Symptom anzusteigen, was die verschiedenen zeitlichen Verläufe des Beginns der Ansammlung von Tränen am Lidrand (subjektive Empfindung) und des tatsächlichen Ausflusses (objektive Bewertung) widerspiegelt. Trotzdem wurde eine gute Korrelation zwischen subjektiven und objektiven Werten für die Tränenbildung im gleichen Probanden gefunden (wobei der Spearman'sche Koeffizient r 0,79 und p kleiner als 0,001 waren), was die allgemeine Verläßlichkeit der Auswertung zeigt.

Nach der Behandlung mit Furosemid schienen die meisten subjektiven und objektiven Symptomwerte im Vergleich zum Plazebo vermindert zu sein, obwohl die ungleichmäßige Verteilung der verschiedenen Symptome und der zeitlichen Festlegung unter den Probanden die statistische Auswertung der Unterschiede wegen des Vorhandenseins mehrerer Ranggleichheiten in jeder Gruppe stark limitiert. Trotzdem zeigt der Wilcoxon- Rangtest eine signifikante Verminderung der Werte in bezug auf Pruritus und Tränenbildung bei 15 bzw. 20 Minuten.

Um das Problem der ungleichmäßigen Symptomverteilung zu umgehen, wurde der Symptommittelwert als Durchschnitt aller objektiven bzw. subjektiven Werte zum jeweiligen Zeitpunkt ausgewertet (Tabelle IV, Abbildung 11 und 12). Die Varianzanalyse der subjektiven Gesamtwerte zeigte einen signifikanten Unterschied zwischen den beiden Behandlungsformen unabhängig von der Zeit (p kleiner als 0,02). Mit dem Wilcoxon-Test wurde eine signifikante Abnahme des subjektiven Gesamtwertes nach Furosemid 10, 15 und 20 Minuten nach der Exposition beobachtet; dasselbe gilt auch für den Maximalwert. Demgegenüber wurden signifikante Unterschiede zwischen objektiven Gesamtwerten nach Furosemid oder Plazebo zu keinem Zeitpunkt gefunden, obwohl die Varianzanalyse auf einen signifikanten Unterschied zwischen den Behandlungsformen hinwies (p kleiner als 0,01).

Um die Hauptsymptome, die für den Schutzeffekt von Furosemid auf die subjektiven Symptomwerte verantwortlich sind, auszusondern und gleichzeitig die Probleme auf Grund der ungleichmäßigen zeitlichen Festlegung der Symptome bei verschiedenen Probanden zu vermeiden, wurde der Mittelwert für jeden Probanden zwischen 5 und 20 Minuten nach der Exposition ausgewertet, da die Varianzanalyse keine signifikanten Unterschiede der Mittelwerte während dieses Zeitraums ergab (Abbildung 13). Ein signifikanter Unterschied wurde zwischen den Mittelwerten für Jucken und Tränenbildung nach Furosemid bzw. Plazebo gefunden, während sich keine signifikanten Unterschiede für alle anderen objektiven und subjektiven Symptomwerte zeigten.

Da die Variabilität der objektiven Werte hauptsächlich auf einen einzelnen Patienten (Nr.5) zurückzuführen war, wurde die Analyse unter Ausschluß des Ausreißers von den Berechnungen wiederholt. Der Wilcoxon-Test für gepaarte Stichproben wies dann auf einen signifikanten Unterschied sowohl von subjektiven (p größer als 0,02) als auch objektiven (p größer als 0,05) Werten hin. Der Effekt auf objektive Werte schien vor allem mit einem Effekt auf Erythem und vermehrte Gefäßzeichnung nach 15 bzw. 20 Minuten nach der Exposition in Verbindung zu stehen (p kleiner als 0,05).

Schließlich wurden Diapositive untersucht, die während der Exposition gemacht worden waren, wobei

die objektiven Werte zum gleichen Zeitpunkt aufgenommen worden waren, um die objektiven Symptomwerte zu bestätigen. Insgesamt 48 Bilder im Verhältnis zu 24 verschiedenen Konjunktivalexpositionen wurden von 3 unabhängigen Betrachtern untersucht, die jedem Bild einen einzelnen Gesamtwert zuordneten. Die Ableseergebnisse der 3 Betrachter korrelierten gut, der Spearman'sche Korrelationskoeffizient schwankte in dem 3-paarigen Vergleich zwischen 0,83 und 0,90 (p kleiner als $10^{-6}$). Wenn der Bildmittelwert mit dem während des gleichen Tests aufgenommenen objektiven Wert verglichen wurde, wurde eine signifikante positive Korrelation mit dem mittleren objektiven Wert beobachtet (r beträgt 0,636, p kleiner als 0,001). Unter den verschiedenen während der Exposition aufgenommenen Parametern war die vermehrte Gefäßzeichnung die mit den vom Bild abgelesenen Werten mehr korrelierende (r beträgt 0,639, p kleiner als 0,001). Ein vollständiger Bildsatz mit sowohl dem Plazebo- als auch dem Furosemid-Test stand in 8 Fällen zur Verfügung. In dieser Gruppe wurden keine signifikanten Unterschiede zwischen den beiden Behandlungsformen bei den Bildwerten gefunden.

## Diskussion

Der Konjunktivaltest war in seiner Verwendung zur Diagnose von Allergien im Laufe der Jahre weit verbreitet und wird als für klinische Untersuchungen geeignet erachtet (Moller C, Bjiorksten B, Nilsson G, Dreborg S. The precision of conjunctival provocation test [Die Genauigkeit des Konjunktivalreiztests]. Allergy 1984, 39:37-41) und auch zur Untersuchung der pathogenen Mechanismen der allergischen Entzündung im Auge (Bonini S, Bonini S, Vecchione A et al. Inflammatory changes in conjunctival scrapings after allergen provocation in humans [Entzündliche Veränderungen in Bindehautsabstrichen nach einer Reizung mit Allergen beim Menschen]. J Allergy Clin Immunol 1988, 82: 462-9; Bonini S, Bonini S, Berruto A, Tomassini M, Carlesimo S, Bucci MG, Balsano F. Conjunctival provocation test as a model for the study of allergy and inflammation in humans [Der konjunktivale Reizungstest als ein Modell für die Untersuchung von Allergie und Entzündung beim Menschen]. Int Arch Allergy Appll Immunol 1989, 88:144-148). Gemäß der vorliegenden Erfindung haben wir den spezifischen Konjunktivaltest benutzt, um den potentiellen therapeutischen Wert einer Lokalbehandlung mit Furosemid bei allergischer Konjunktivitis zu bewerten. Die Auswertung des Tests hat sich trotz des Mangels an direkten Meßvariablen und, obwohl die beträchtliche Variabilität zwischen den Probanden beim Vergleich homogener Gruppen einige Schwierigkeiten verursacht, als relativ reproduzierbar erwiesen. Eine Lokalbehandlung mit Furosemid verminderte signifikant sowohl objektive als auch subjektive Werte im Vergleich zum Plazebo, obwohl der Effekt bei einigen der letzteren deutlicher zu sein schien als bei objektiven Entzündungssymptomen, was wahrscheinlich die größere Variabilität dieser Parameter widerspiegelt. Die erfindungsgemäßen Daten zeigen, daß Furosemid einen Schutzeffekt bei der spezifischen Allergenexposition der Bindehaut ausübt.

Zusammengefaßt zeigen die Daten, daß eine Lokalbehandlung mit Furosemid sehr gut verträglich ist und die durch eine spezifische Allergenexposition hervorgerufene Konjunktivalreaktion bei Probanden mit saisonaler allergischer Konjunktivitis signifikant reduziert. Diese Ergebnisse zeigen, daß Furosemid für die Behandlung der allergischen Erkrankungen des Auges potentiell wertvoll ist.

Tabelle I - Klinische Merkmale der untersuchten Patienten

| N | Geschlecht | Alter | Symptome | verwendetes Allergen[1)] | Dosis (AUR) | andere Hautreaktionen[1)] |
|---|---|---|---|---|---|---|
| 1 | W | 45 | Saisonale Rhinokonjunktivitis | Pa | 32 | |
| 2 | M | 18 | Saisonale Rhinokonjunktivitis | Gr | 32 | |
| 3 | W | 23 | Saisonale Rhinokonjunktivitis | Pa | 100 | Gr DP |
| 4 | M | 17 | Saisonale Rhinokonjunktivitis | Gr | 100 | |
| 5 | M | 37 | Saisonale Rhinokonjunktivitis | Gr | 320 | Pa, Ol, Cy |
| 6 | M | 27 | Saisonale Rhinokonjunktivitis | Gr | 100 | |
| 7 | M | 49 | Saisonale Rhinokonjunktivitis und Asthma | Gr | 32 | Ol, Comp |
| 8 | M | 18 | Saisonale Rhinokonjunktivitis | Gr | 10 | |
| 9 | W | 12 | Saisonale Rhinokonjunktivitis und Asthma | Gr | 100 | |
| 10 | M | 22 | Saisonale Rhinokonjunktivitis | Gr | 32 | Pa |

1) Gr: Graspollen, Pa: Parietaria officinalis
Ol: Ölbaum Cy: Zypresse
Comp: Compositae DP: Dermatophagoides pteronissimus

Tabelle II - Der Effekt von Furosemid auf subjektive Werte nach einer Allergenexposition

JUCKEN

| | Plazebo | | | Furosemid | | |
|---|---|---|---|---|---|---|
| ZEIT | Median | Mittelwert | ± SE | Median | Mittelwert | ± SE |
| 0 | 0,0 | 0,0 | ± 0,0 | 0,0 | 0,00 | ± 0,0 |
| 5′ | 14,0 | 27,80 | ± 9,05 | 14,0 | 19,50 | ± 5,26 |
| 10′ | 11,0 | 18,50 | ± 5,69 | 7,0 | 12,00 | ± 4,94 |
| 15′ | 10,0 | 14,70 | ± 4,65 | 7,0 | 10,10 | ± 3,48 |
| 20′ | 12,0 | 15,60 | ± 5,15 | 3,0 | 8,20 | ± 3,95 |
| Mittelwert[a] | 11,0 | 15,40 | ± 4,17 | 6,0 | 10,00 | ± 3,12 |
| Max[b] | 20,0 | 30,50 | ± 9,29 | 14,0 | 22,10 | ± 5,53 |

BRENNEN

| | Plazebo | | | Furosemid | | |
|---|---|---|---|---|---|---|
| ZEIT | Median | Mittelwert | ± SE | Median | Mittelwert | ± SE |
| 0 | 0,0 | 0,0 | ± 0,0 | 0,0 | 0,00 | ± 0,0 |
| 5′ | 8,0 | 18,50 | ± 7,37 | 1,5 | 5,80 | ± 2,61 |
| 10′ | 2,5 | 11,80 | ± 4,67 | 0,0 | 7,90 | ± 4,26 |
| 15′ | 1,5 | 12,60 | ± 5,60 | 0,0 | 9,40 | ± 6,24 |
| 20′ | 1,0 | 12,90 | ± 6,20 | 0,0 | 10,90 | ± 7,38 |
| Mittelwert | 6,0 | 11,10 | ± 4,29 | 1,0 | 6,70 | ± 3,60 |
| Max | 12,0 | 22,70 | ± 8,00 | 6,0 | 15,20 | ± 7,04 |

Tabelle II - Forts.

PHOTOPHOBIE

| | Plazebo | | | Furosemid | | |
|---|---|---|---|---|---|---|
| ZEIT | Median | Mittelwert | ± SE | Median | Mittelwert | ± SE |
| 0 | 0,0 | 0,0 | ± 0,0 | 0,0 | 0,00 | ± 0,0 |
| 5′ | 0,0 | 10,10 | ± 5,96 | 0,0 | 4,10 | ± 2,16 |
| 10′ | 1,0 | 6,00 | ± 3,44 | 0,0 | 4,80 | ± 3,08 |
| 15′ | 1,0 | 7,20 | ± 4,54 | 0,0 | 3,10 | ± 2,19 |
| 20′ | 1,0 | 6,60 | ± 5,08 | 0,0 | 3,30 | ± 2,89 |
| Mittelwert | 1,5 | 6,00 | ± 3,42 | 0,5 | 3,20 | ± 1,89 |
| Max | 4,5 | 13,30 | ± 6,29 | 1,5 | 6,40 | ± 3,25 |

TRÄNENBILDUNG

| | Plazebo | | | Furosemid | | |
|---|---|---|---|---|---|---|
| ZEIT | Median | Mittelwert | ± SE | Median | Mittelwert | ± SE |
| 0 | 0,0 | 0,0 | ± 0,0 | 0,0 | 0,00 | ± 0,0 |
| 5′ | 6,0 | 10,20 | ± 3,84 | 2,5 | 6,80 | ± 2,60 |
| 10′ | 7,5 | 12,50 | ± 4,78 | 1,0 | 9,10 | ± 3,72 |
| 15′ | 7,5 | 16,30 | ± 7,49 | 0,0 | 10,20 | ± 6,59 |
| 20′ | 4,5 | 18,60 | ± 8,96 | 0,0 | 11,40 | ± 7,72 |
| Mittelwert | 4,5 | 11,40 | ± 4,81 | 1,0 | 7,40 | ± 3,59 |
| Max | 13,5 | 21,70 | ± 8,43 | 3,5 | 16,40 | ± 7,48 |

Anmerkungen zu Tabelle II:

a) Mittelwert der zwischen 5 und 20 Minuten nach der Exposition aufgenommenen Werte.

b) Maximalwert der zwischen 5 und 20 Minuten nach der Exposition aufgenommenen Werte.

Tabelle III - Effekt von Furosemid auf objektive Werte nach einer Allergenexposition

ERYTHEM

| | Plazebo | | | Furosemid | | |
|---|---|---|---|---|---|---|
| ZEIT | Median | Mittelwert | ± SE | Median | Mittelwert | ± SE |
| 0 | 0,0 | 0,0 | ± 0,0 | 0,0 | 0,00 | ± 0,0 |
| 5′ | 17,5 | 19,50 | ± 4,92 | 10,0 | 13,20 | ± 3,24 |
| 10′ | 18,5 | 22,80 | ± 5,31 | 11,0 | 14,00 | ± 3,09 |
| 15′ | 20,5 | 24,60 | ± 5,71 | 10,0 | 18,60 | ± 6,76 |
| 20′ | 27,5 | 28,30 | ± 5,93 | 8,5 | 17,80 | ± 6,46 |
| Mittelwert | 15,5 | 18,90 | ± 4,06 | 8,5 | 12,60 | ± 3,32 |
| Max | 27,5 | 31,20 | ± 6,18 | 19,0 | 25,20 | ± 6,33 |

OEDEM

| | Plazebo | | | Furosemid | | |
|---|---|---|---|---|---|---|
| ZEIT | Median | Mittelwert | ± SE | Median | Mittelwert | ± SE |
| 0 | 0,0 | 0,0 | ± 0,0 | 0,0 | 0,00 | ± 0,0 |
| 5′ | 2,0 | 5,70 | ± 2,34 | 1,0 | 2,50 | ± 0,96 |
| 10′ | 5,0 | 7,50 | ± 2,13 | 1,5 | 3,90 | ± 1,73 |
| 15′ | 4,0 | 6,70 | ± 2,39 | 1,0 | 7,10 | ± 3,67 |
| 20′ | 3,5 | 5,80 | ± 2,04 | 0,0 | 8,30 | ± 5,78 |
| Mittelwert | 3,0 | 5,20 | ± 1,71 | 2,0 | 4,40 | ± 2,15 |
| Max | 5,5 | 8,40 | ± 2,18 | 5,0 | 10,6 | ± 5,71 |

Tabelle III - Forts.

vermehrte Gefäßzeichnung

| | Plazebo | | | Furosemid | | |
|---|---|---|---|---|---|---|
| ZEIT | Median | Mittelwert | ± SE | Median | Mittelwert | ± SE |
| 0 | 0,0 | 0,0 | ± 0,0 | 0,0 | 0,00 | ± 0,0 |
| 5′ | 26,5 | 27,80 | ± 4,58 | 18,0 | 21,80 | ± 2,98 |
| 10′ | 30,5 | 31,70 | ± 3,61 | 21,0 | 22,60 | ± 4,46 |
| 15′ | 35,0 | 32,50 | ± 5,19 | 18,5 | 22,80 | ± 5,97 |
| 20′ | 35,5 | 35,50 | ± 5,32 | 21,5 | 23,60 | ± 6,77 |
| Mittelwert | 27,0 | 25,60 | ± 3,17 | 14,5 | 18,20 | ± 3,54 |
| Max | 38,0 | 40,70 | ± 5,17 | 31,0 | 31,70 | ± 5,17 |

TRÄNENBILDUNG

| | Plazebo | | | Furosemid | | |
|---|---|---|---|---|---|---|
| ZEIT | Median | Mittelwert | ± SE | Median | Mittelwert | ± SE |
| 0 | 0,0 | 0,0 | ± 0,0 | 0,0 | 0,00 | ± 0,0 |
| 5′ | 11,5 | 10,00 | ± 2,42 | 3,0 | 8,20 | ± 4,11 |
| 10′ | 9,0 | 11,60 | ± 3,15 | 2,0 | 7,20 | ± 4,83 |
| 15′ | 3,0 | 10,20 | ± 4,36 | 0,5 | 9,20 | ± 6,68 |
| 20′ | 5,5 | 15,00 | ± 5,98 | 2,0 | 7,00 | ± 4,22 |
| Mittelwert | 7,0 | 9,30 | ± 2,74 | 1,5 | 6,30 | ± 3,86 |
| Max | 14,0 | 19,50 | ± 5,69 | 6,5 | 12,40 | ± 6,37 |

**Tabelle IV - Effekt von Furosemid auf die Gesamtmittelwerte nach einer Allergenexposition**

**SUBJEKTIVER GESAMTWERT**

| | Plazebo | | | Furosemid | | |
|---|---|---|---|---|---|---|
| ZEIT | Median | Mittelwert | ± SE | Median | Mittelwert | ± SE |
| 0 | 0,0 | 0,0 | ± 0,0 | 0,0 | 0,00 | ± 0,0 |
| 5' | 12,0 | 16,70 | ± 5,40 | 7,0 | 8,90 | ± 1,89 |
| 10' | 9,0 | 12,20 | ± 3,62 | 6,5 | 8,50 | ± 3,20 |
| 15' | 7,0 | 12,80 | ± 4,52 | 2,0 | 8,30 | ± 3,62 |
| 20' | 5,5 | 13,50 | ± 5,39 | 0,5 | 8,40 | ± 4,90 |
| Mittelwert | 7,5 | 11,00 | ± 3,38 | 3,0 | 6,70 | ± 2,47 |
| Max | 12,5 | 20,90 | ± 5,96 | 8,0 | 13,60 | ± 4,29 |

**OBJEKTIVER GESAMTWERT**

| | Plazebo | | | Furosemid | | |
|---|---|---|---|---|---|---|
| ZEIT | Median | Mittelwert | ± SE | Median | Mittelwert | ± SE |
| 0 | 0,0 | 0,0 | ± 0,0 | 0,0 | 0,00 | ± 0,0 |
| 5' | 13,5 | 15,80 | ± 2,88 | 9,0 | 11,40 | ± 2,09 |
| 10' | 16,5 | 18,40 | ± 2,88 | 8,5 | 12,00 | ± 3,01 |
| 15' | 20,0 | 18,50 | ± 3,58 | 6,5 | 14,40 | ± 5,35 |
| 20' | 19,5 | 21,20 | ± 4,08 | 8,5 | 14,20 | ± 5,38 |
| Mittelwert | 14,0 | 14,80 | ± 2,58 | 7,0 | 10,30 | ± 2,90 |
| Max | 20,0 | 22,40 | ± 4,09 | 13,0 | 18,70 | ± 4,91 |

**Legenden zu den Abbildungen**

Abbildungen 1-10. Einzelwerte der während einer spezifischen Allergenexposition aufgenommenen subjektiven (links) und objektiven (rechts) Symptomwerte nach einer Behandlung mit Plazebo (leere

Zeichen, durchgezogene Linien) bzw. Furosemid (ausgefüllte Zeichen, gepunktete Linien) für die jeweiligen Patienten Nr. 1 - Nr. 10. Subjektive Symptome: Jucken (Kreis), Brennen (Raute), Photophobie (Dreieck), Tränenbildung (umgekehrtes Dreieck). Objektive Symptome: Erythem (Kreis), Ödem (Raute), vermehrte Gefäßzeichnung (Dreieck), Tränenbildung (umgekehrtes Dreieck); die Werte sind gegen die Zeit nach der Exposition aufgetragen.

Abbildung 11. Mittelwert und SE von während der Exposition aufgenommenen subjektiven Gesamtwerten nach einer Behandlung mit Plazebo (leere Zeichen) bzw. Furosemid (ausgefüllte Zeichen). Eine Varianzanalyse zeigte einen signifikanten Unterschied zwischen den Behandlungsformen (p kleiner als 0,02), aber keine signifikanten zeitlichen Schwankungen zwischen 5 und 20 Minuten.

Abbildung 12. Mittelwert und SE von während der Exposition aufgenommenen objektiven Gesamtwerten nach einer Behandlung mit Plazebo (leere Zeichen) bzw. Furosemid (ausgefüllte Zeichen). Eine Varianzanalyse zeigte einen signifikanten Unterschied zwischen den Behandlungsformen (p kleiner als 0,01), aber keine signifikanten zeitlichen Schwankungen zwischen 5 und 20 Minuten.

Abbildung 13. Median des zwischen 5 und 20 Minuten nach einer Exposition aufgenommenen mittleren Wertes für jeden subjektiven und objektiven Parameter noch einer Behandlung mit Plazebo (leere Balken) bzw. Furosemid (ausgefüllt). *) p kleiner als 0,05, Wilcoxon-Test für gepaarte Stichproben.

**Patentansprüche**

1. Methode zur Behandlung bzw. Verhütung allergischer Konjunktivitis, gekennzeichnet durch die Applikation einer geeigneten Furosemidmenge in den Fornix conjunctivae des Auges.

2. Verwendung von Furosemid zur Behandlung bzw. Verhütung allergischer Konjunktivitis, dadurch gekennzeichnet, durch die Applikation von Furosemid in den Fornix conjunctivae des Auges.

3. Verwendung von Furosemid zur Herstellung eines Medikaments zur Behandlung bzw. Verhütung allergischer Konjunktivitis.

4. Furosemid zur Verwendung in einer Methode zur Behandlung bzw. Verhütung allergischer Konjunktivitis.

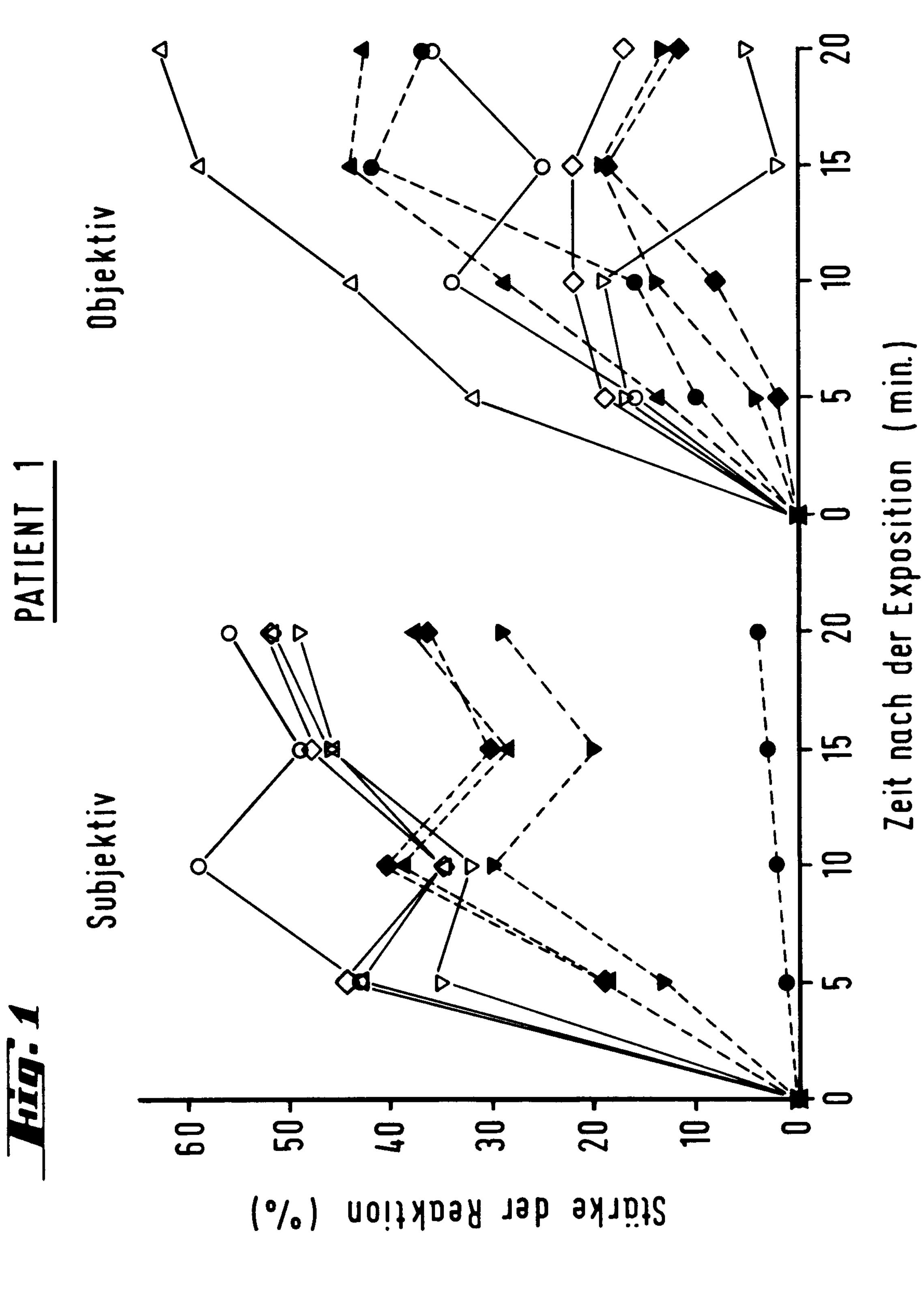
Fig. 1
PATIENT 1
Subjektiv
Objektiv
Stärke der Reaktion (%)
0
10
20
30
40
50
60
Zeit nach der Exposition (min.)
0
5
10
15
20
0
5
10
15
20

Fig. 2

PATIENT 2

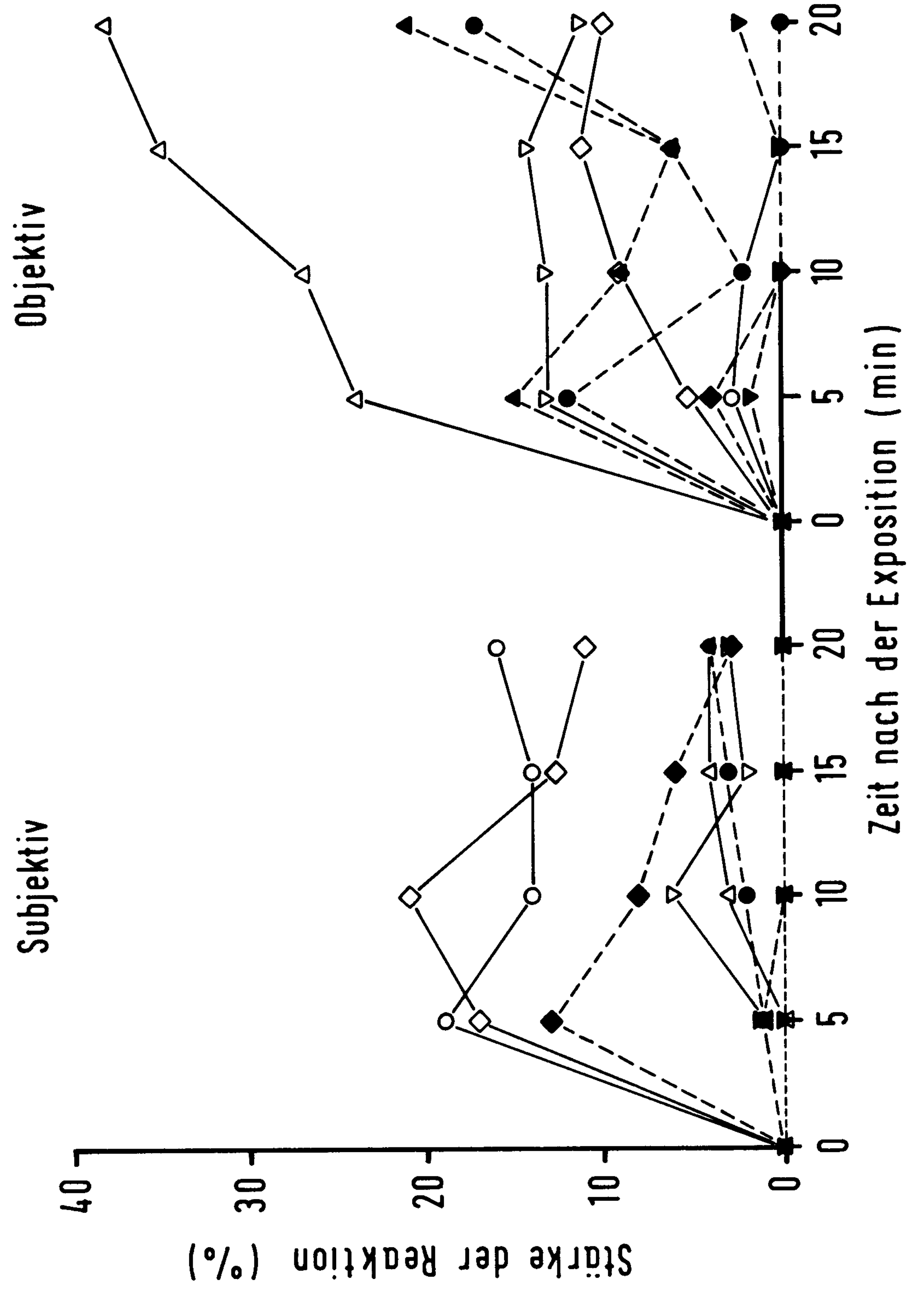

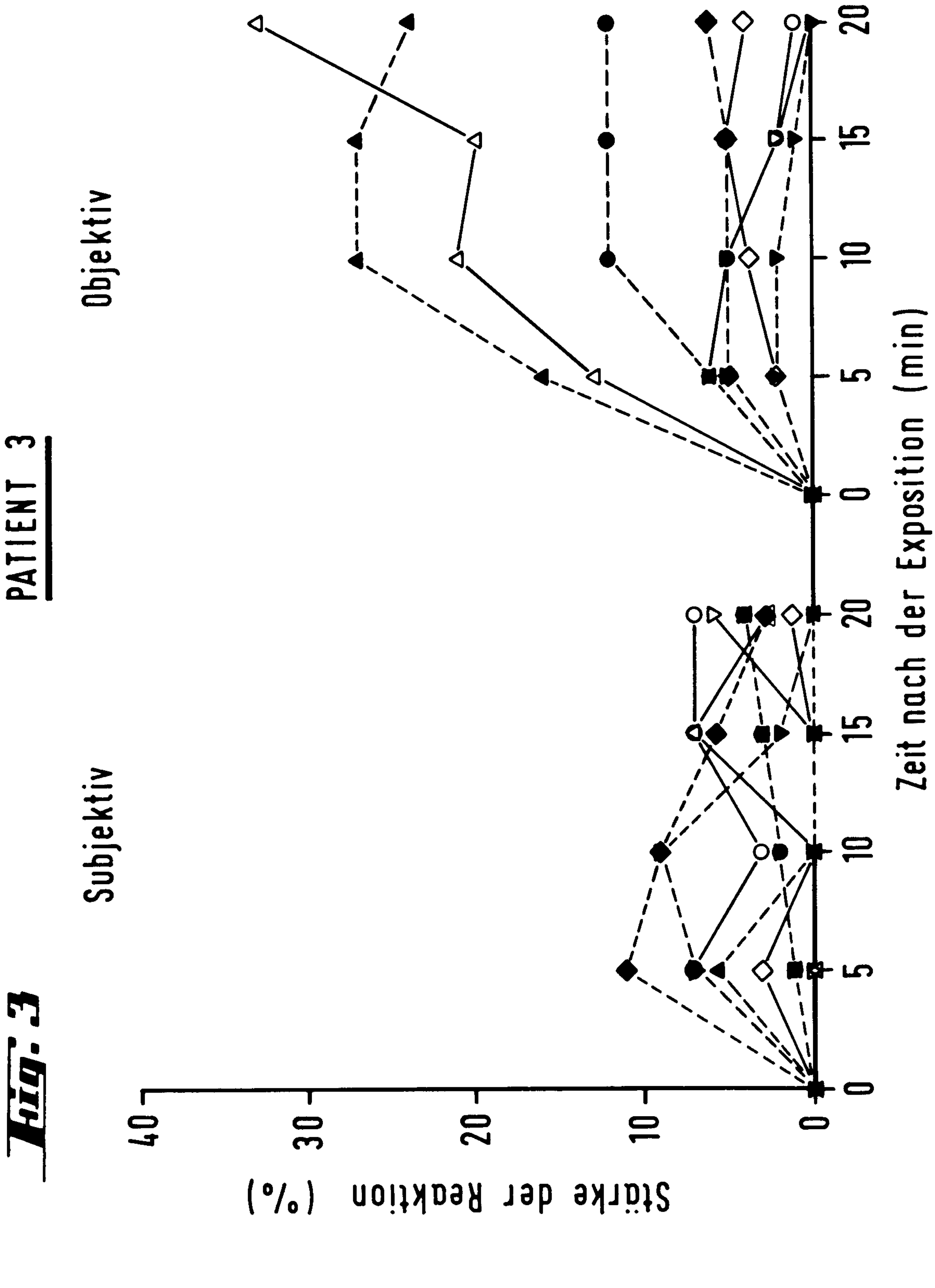
Fig. 3
PATIENT 3
Subjektiv
Objektiv
Stärke der Reaktion (%)
40
30
20
10
0
0
5
10
15
20
0
5
10
15
20
Zeit nach der Exposition (min)

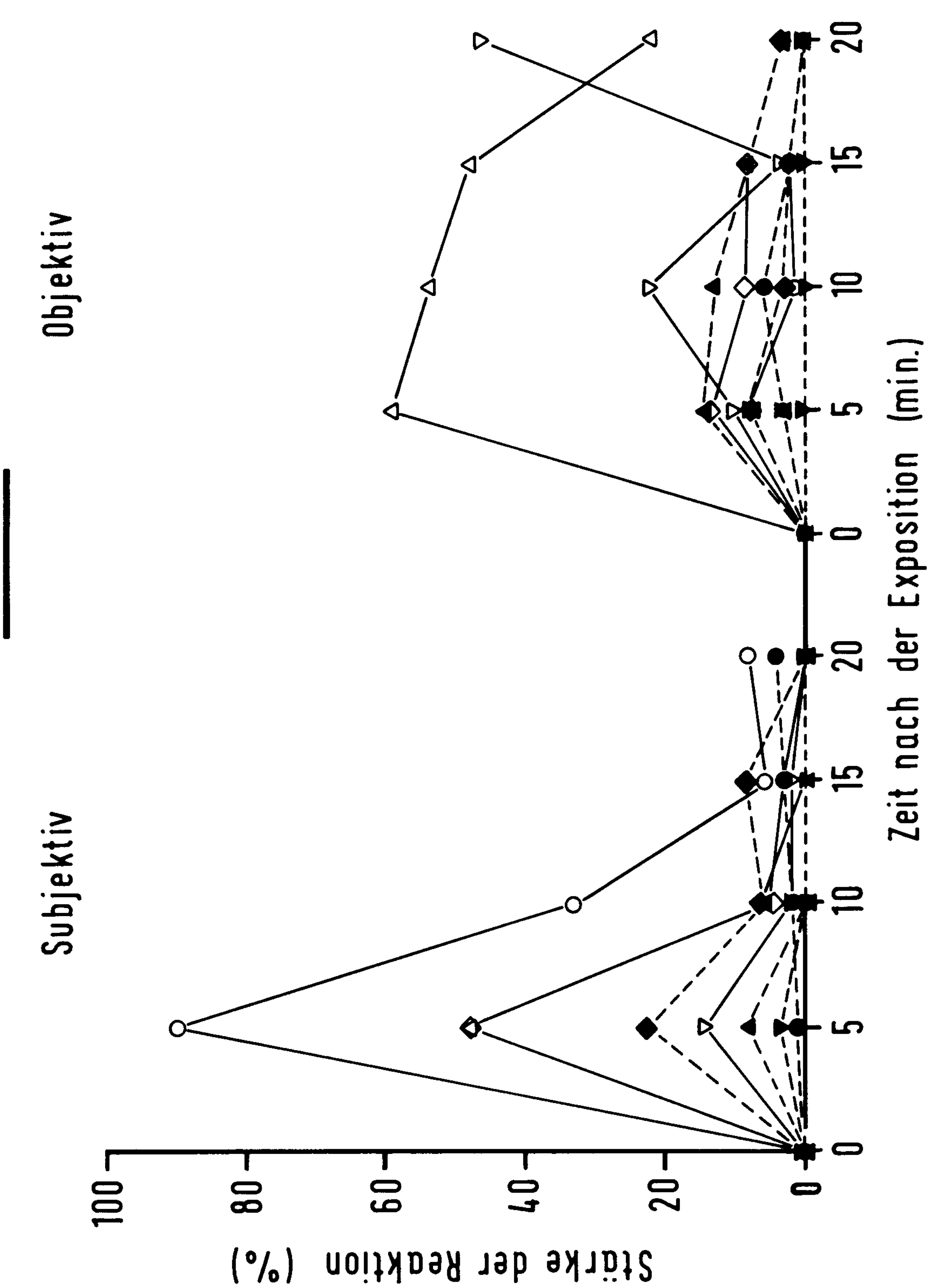
PATIENT 4
Subjektiv
Objektiv
Stärke der Reaktion (%)
Zeit nach der Exposition (min.)
0
20
40
60
80
100
0
5
10
15
20

Fig. 4

Fig. 5

PATIENT 5

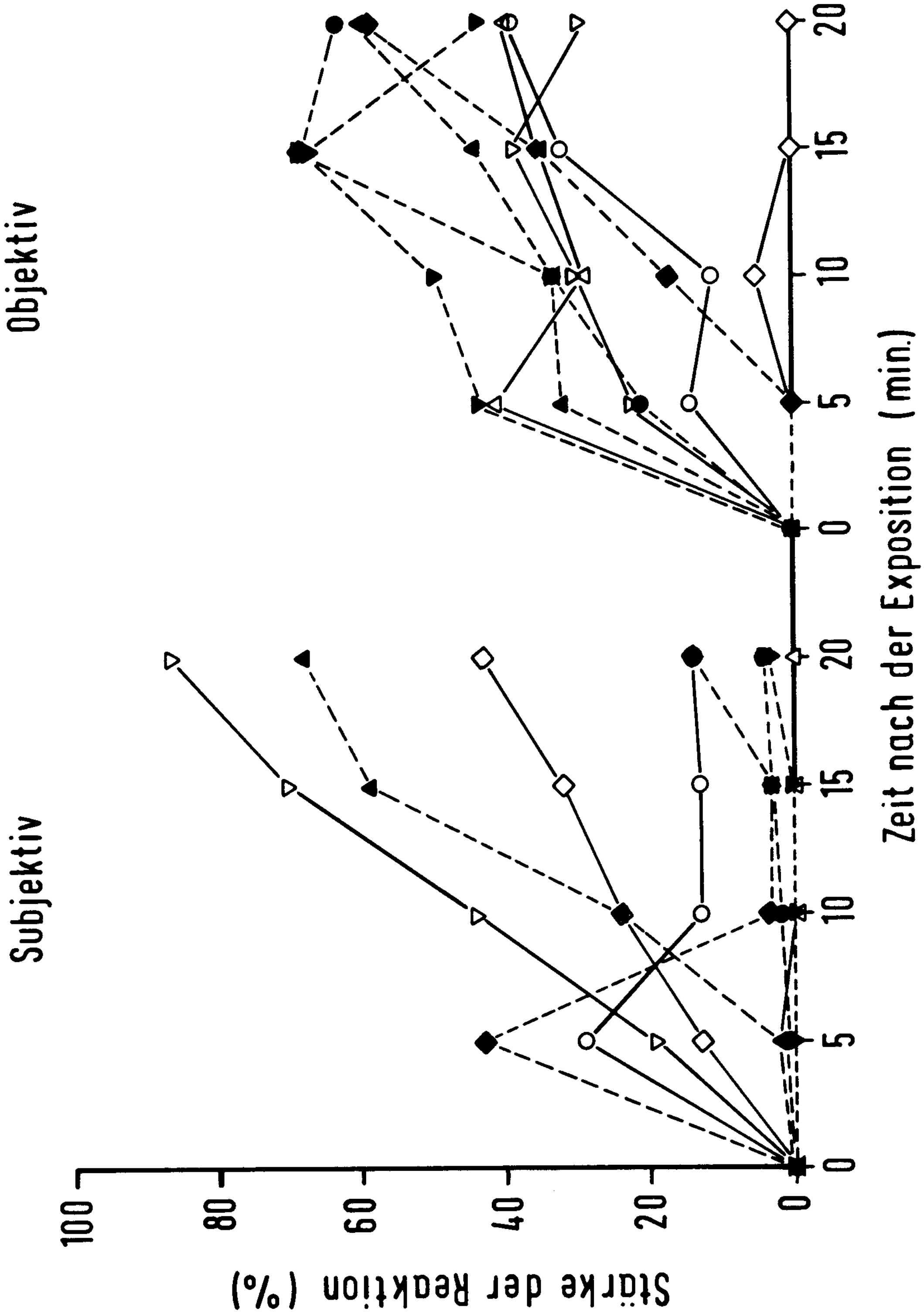

Fig. 6

PATIENT 6

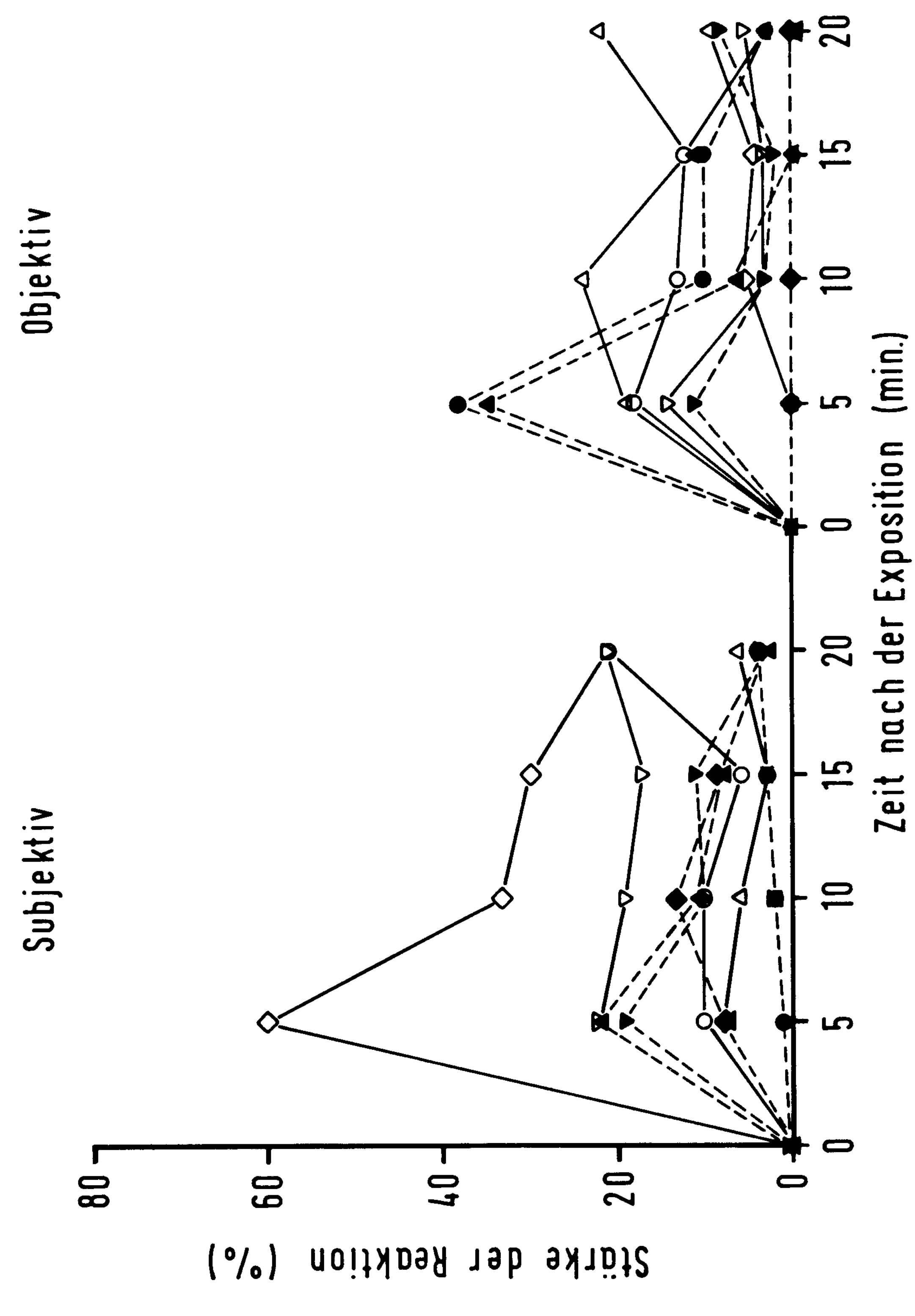

Fig. 7

PATIENT 7

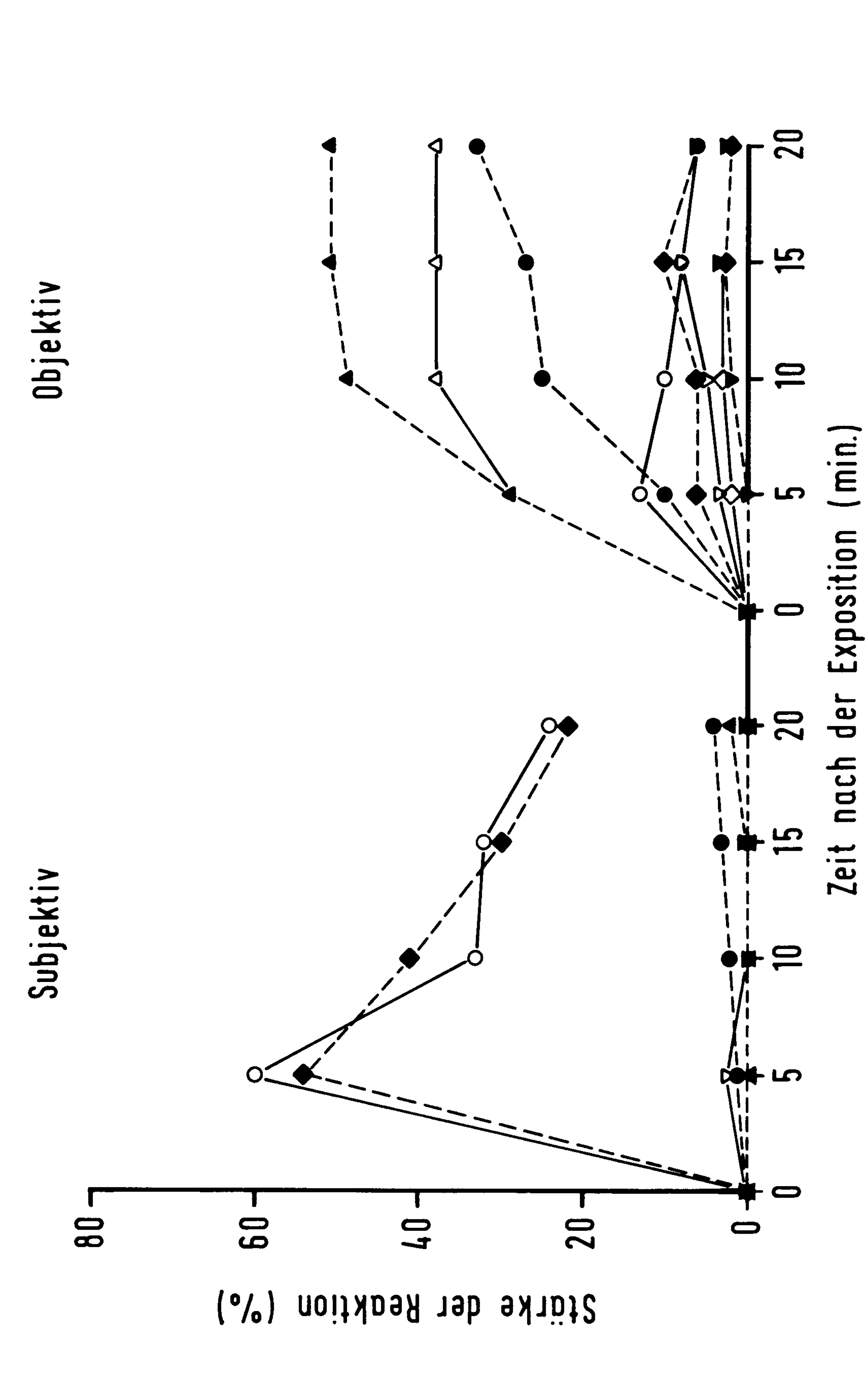

Fig. 8

PATIENT 8

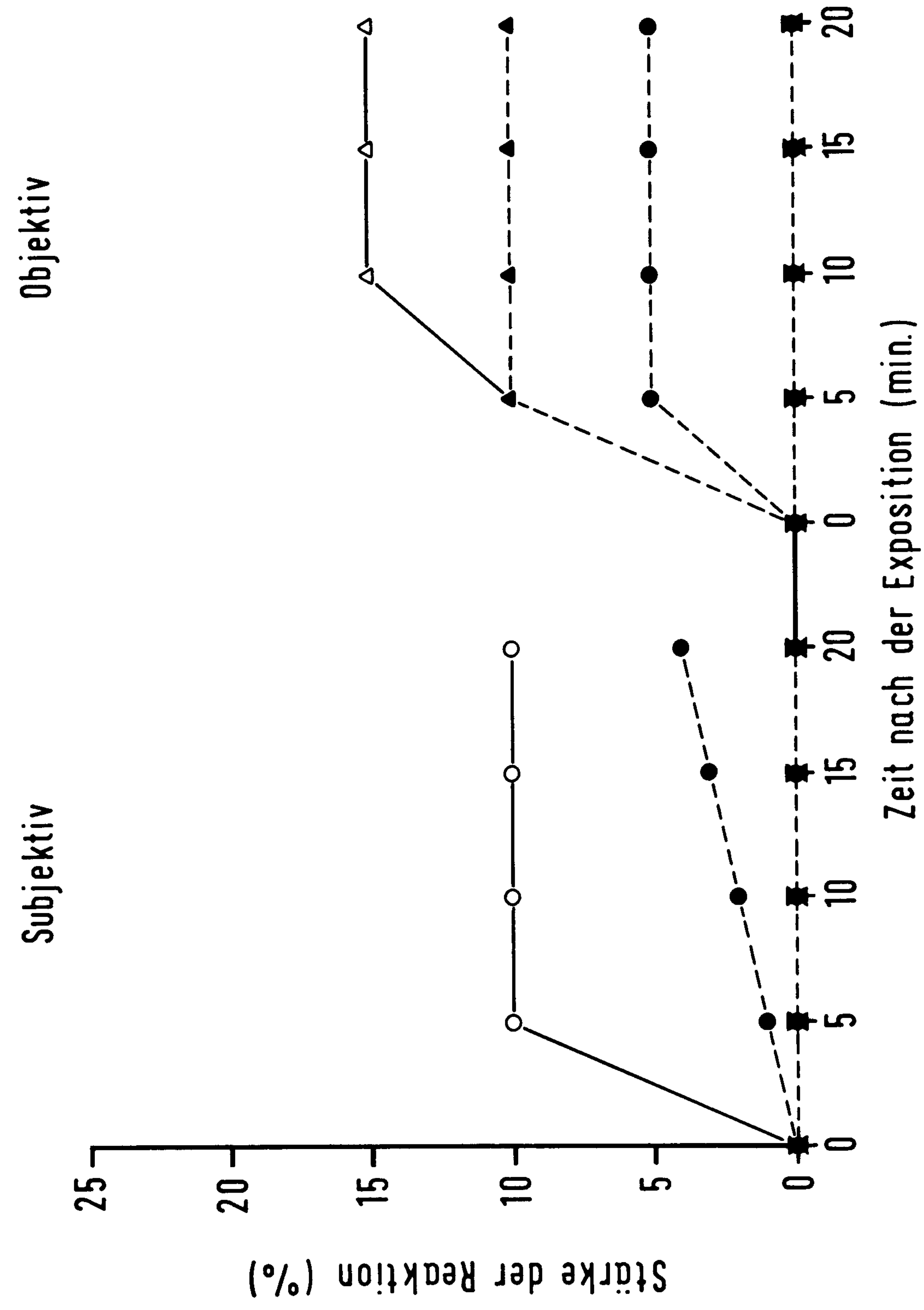

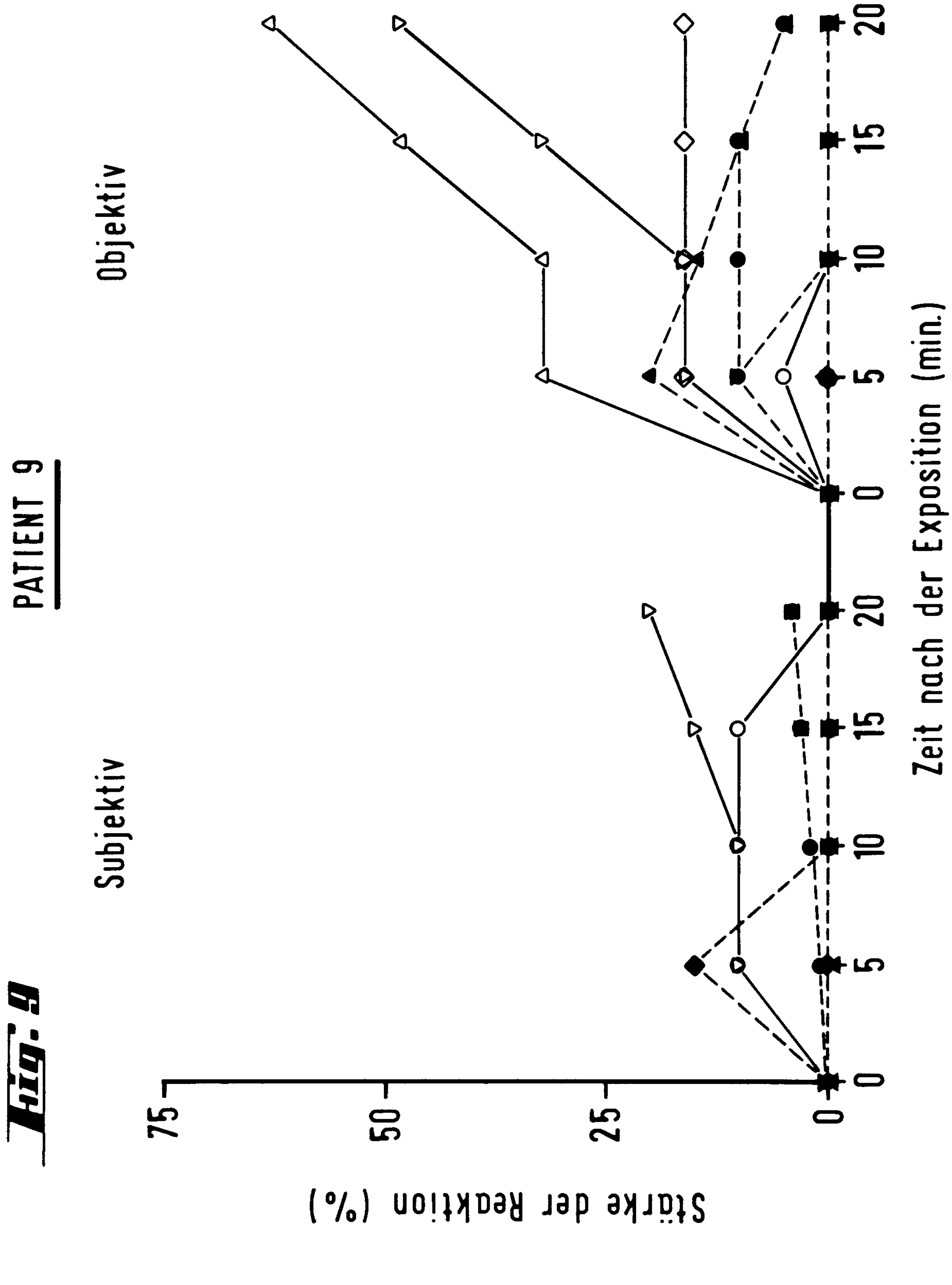
PATIENT 9
Subjektiv
Objektiv
Stärke der Reaktion (%)
0
25
50
75
0
5
10
15
20
0
5
10
15
20
Zeit nach der Exposition (min.)

Fig. 9

Fig. 10

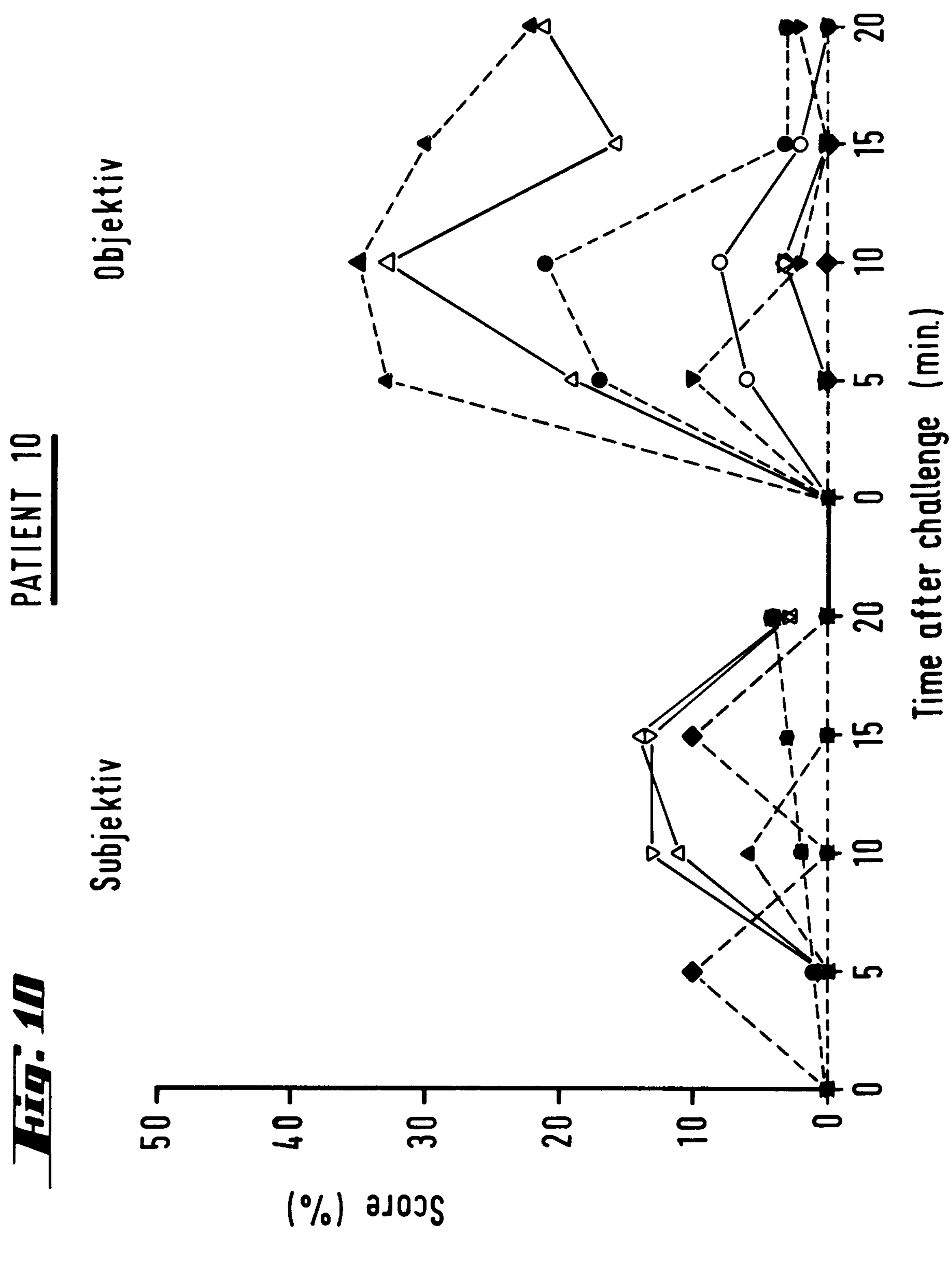
PATIENT 10
Subjektiv
Objektiv
Score (%)
0
10
20
30
40
50
0
5
10
15
20
0
5
10
15
20
Time after challenge (min.)

Fig. 11

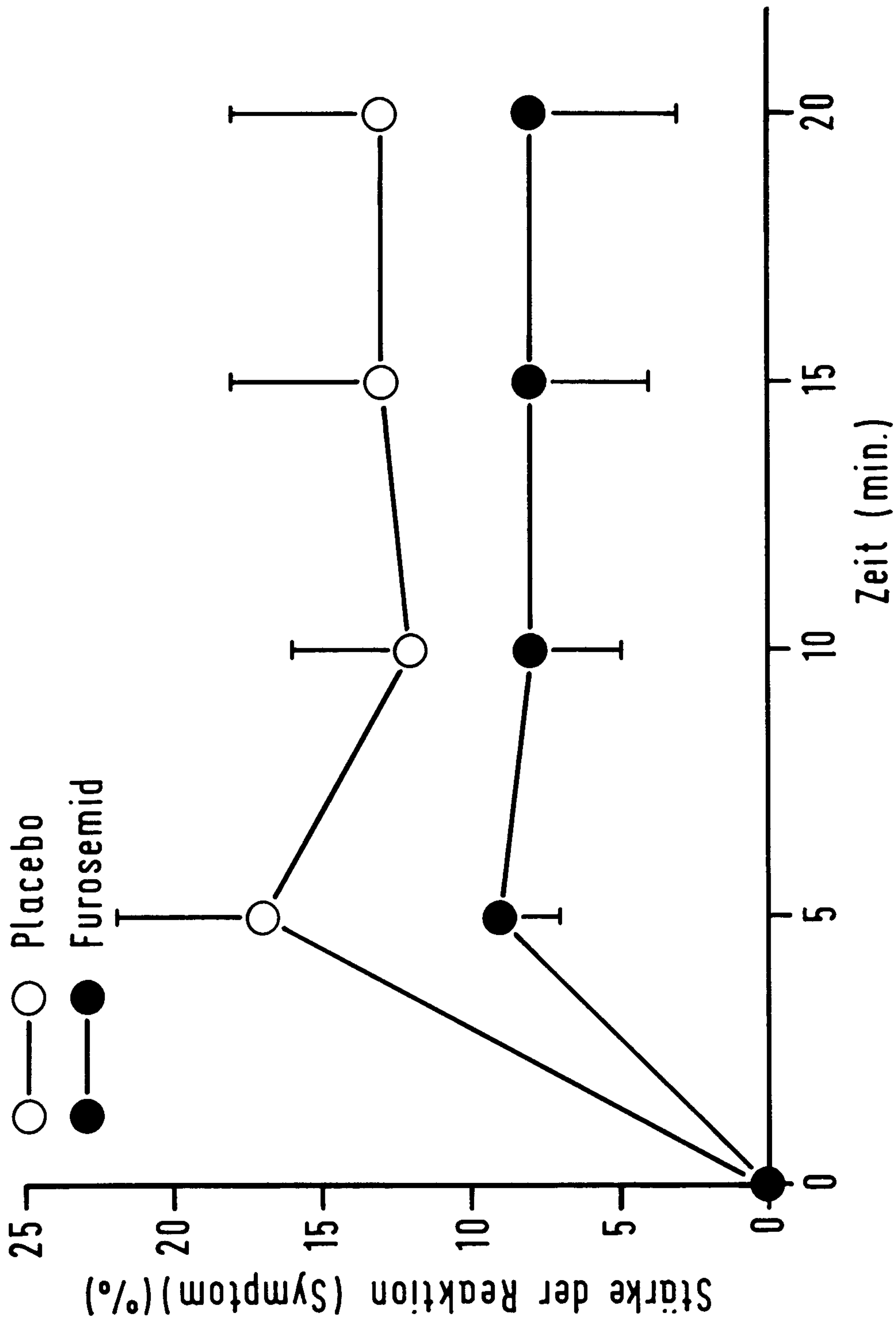
Placebo
Furosemid
Stärke der Reaktion (Symptom) (%)
25
20
15
10
5
0
0
5
10
15
20
Zeit (min.)

Fig. 12

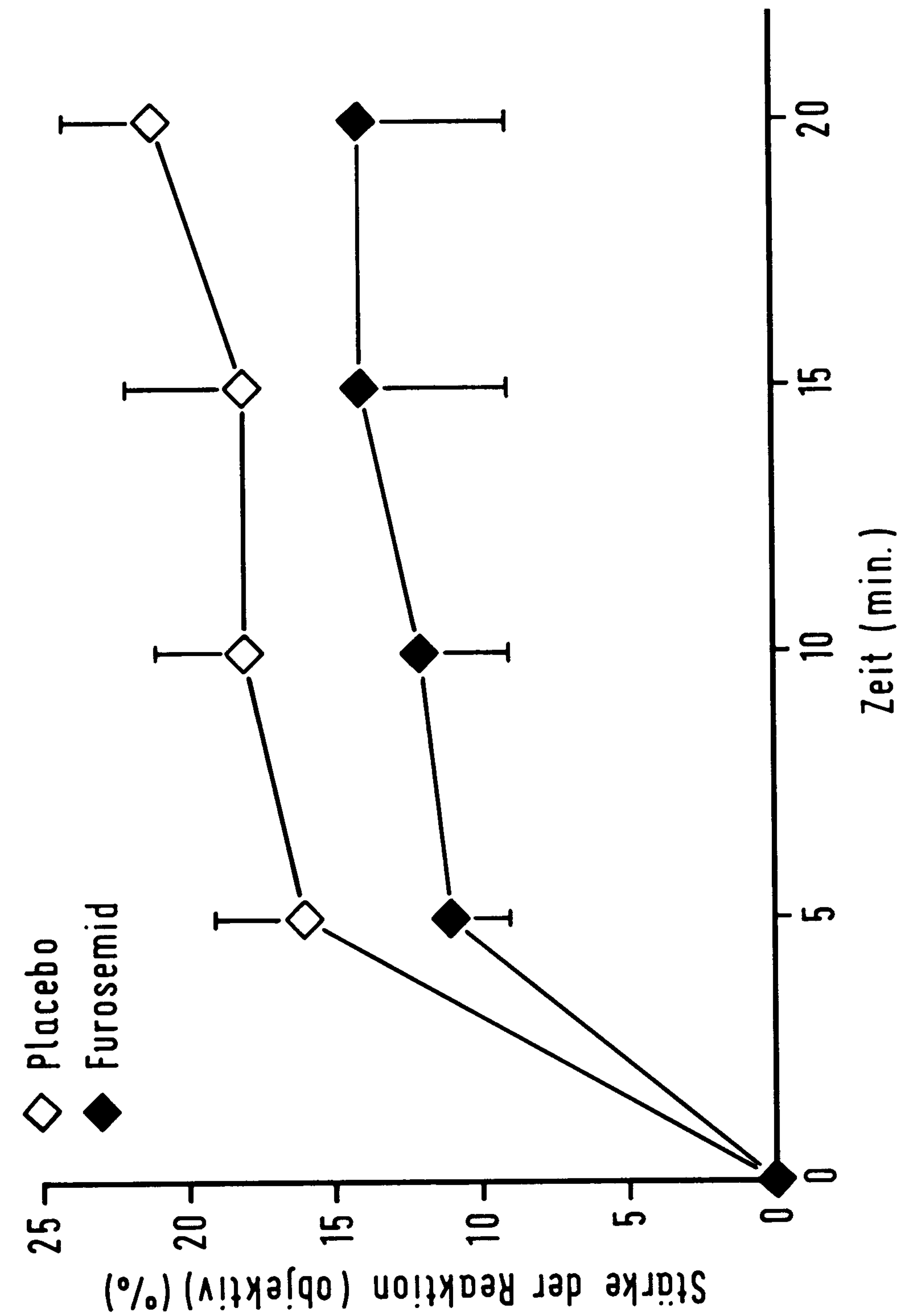
Placebo
Furosemid
Stärke der Reaktion (objektiv) (%)
25
20
15
10
5
0
Zeit (min.)
0
5
10
15
20

Fig. 13

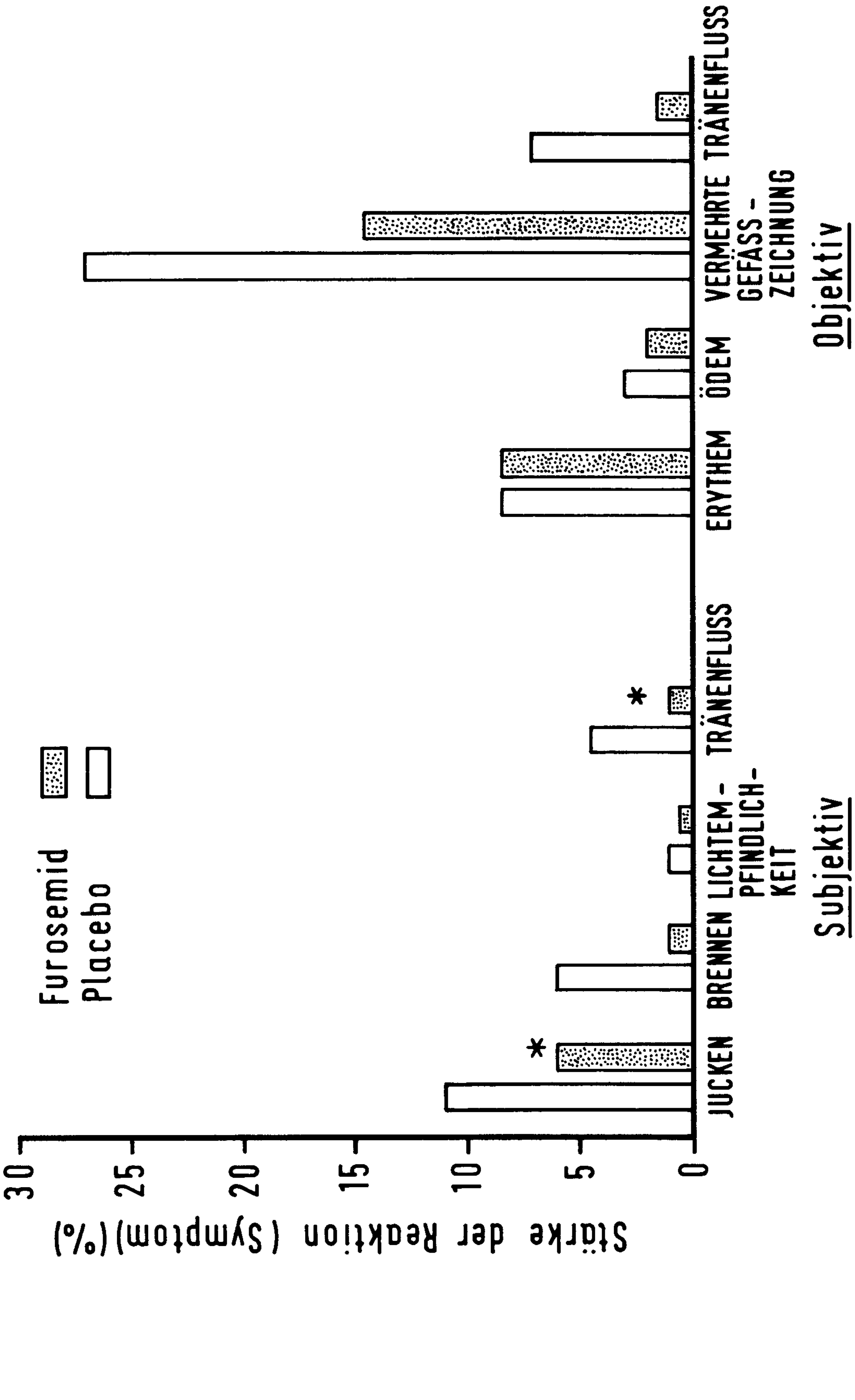
Furosemid
Placebo
Stärke der Reaktion (Symptom)(%)
0
5
10
15
20
25
30
*
*
JUCKEN
BRENNEN
LICHTEM-PFINDLICH-KEIT
TRÄNENFLUSS
ERYTHEM
ÖDEM
VERMEHRTE GEFÄSS-ZEICHNUNG
TRÄNENFLUSS
Subjektiv
Objektiv